# EUROPEAN PATENT APPLICATION

(11) **EP 4 144 357 A1**
(43) Date of publication of application: **08.03.2023**
(21) Application number: 21797887.3
(22) Date of filing: 28.04.2021
(51) Int. Cl.: A61K 36/537, A61K 36/258, A61K 31/045, A61K 9/28, A61P 31/14, A61P 11/00

(54) **TRADITIONAL CHINESE MEDICINE COMPOSITION, AND APPLICATION OF PREPARATION OF TRADITIONAL CHINESE MEDICINE COMPOSITION IN PREPARING MEDICINE FOR PREVENTING AND/OR TREATING CORONAVIRUS DISEASE 2019**

(30) Priority: 29.04.2020 CN 202010353323
(71) Applicant: Tasly Pharmaceutical Group Co., Ltd., Tianjin 300410 (CN)
(72) Inventor: SUN, He, Tianjin 300410 (CN); SU, Xuefeng, Tianjin 300410 (CN); ZHONG, Zhiyong, Tianjin 300410 (CN); LI, Anni, Tianjin 300410, China (CN); YAN, Xijun, Tianjin 300410 (CN); WU, Naifeng, Tianjin 300410 (CN); YAN, Kaijing, Tianjin 300410 (CN)
(74) Representative: Gulde & Partner
(86) International application number: PCT/CN2021/090690
(87) International publication number: WO 2021/219031

(57) **Abstract**

The present invention relates to an application of a traditional Chinese medicine composition and formulation thereof in the preparation of medicaments for preventing and/or treating novel coronavirus pneumonia.

## Description

### FIELD OF THE INVENTION

The present invention relates to the field of medicine, particularly relates to an application of a traditional Chinese medicine composition and formulation thereof in the preparation of medicaments for preventing and/or treating novel coronavirus pneumonia.

### BACKGROUND OF THE INVENTION

For Novel coronavirus pneumonia, International Committee on Taxonomy of Viruses named this novel coronavirus severe acute respiratory syndrome coronavirus 2 (SARS-CoV-2). World Health Organization (WHO) announced on the same day that the officially name of the disease caused by this virus, which spreads rapidly, is COVID-19 (coronavirus disease 2019).

Based on current epidemiological investigations, clinical symptoms of COVID-19 patients are mainly fever, fatigue, and dry cough, with a few patients having symptoms such as nasal congestion, runny nose, sore throat, and diarrhea. About half of the patients develop dyspnea and/or hypoxemia after 1 week, and severe cases may rapidly progress to acute respiratory distress syndrome (ARDS), septic shock, and uncorrectable metabolic acid, elevated blood viscosity, and coagulation dysfunction. The treatment of COVID-19 hypoxemia is critical that patients diagnosed with severe disease develop respiratory distress and/or hypoxemia one week after the onset of the disease, and require clinical support for respiratory and circulatory support, aggressive management of complications, treatment of underlying diseases, and prevention of secondary infections based on symptomatic treatment. Compared with patients with mild disease, patients with severe disease have significant interstitial changes in both lungs, with multiple ground glass shadows, infiltration shadows, and pulmonary consolidation; meanwhile, the body's Th1 response is enhanced, the release of pro-inflammatory factors is increased, and inflammation invades the alveolar walls and adjacent alveolar spaces, and progressively causing thickening of alveolar septa and pulmonary fibrosis, accompanied by pulmonary dysfunction and progressive dyspnea, and alveoli cannot realize air exchange process, weakened oxygenation function further leads to a decrease in blood oxygen content, resulting in hypoxemia, severe patients can rapidly progress to acute respiratory distress syndrome (ARDS), septic shock, uncorrectable metabolic acidosis, coagulation dysfunction, and multiple organ failure. In addition, it has been reported that sustained decrease in oxygenation index can be one of the early warning factors for severe and critical cases, and patients with exacerbations have had more than 12 hours of oxygenation index of only 100-150 mmHg. Such patients are highly likely to induce cytokine storm (CSS) because of hypoxia, and CSS will lead to the release of large amounts of inflammatory factors in the lungs and promote inflammatory damage such as pulmonary fibrosis. Therefore, during the treatment of COVID-19, it is especially important to focus on the changes of oxygenation index, blood oxygen saturation and other related indexes of patients, to prevent and control, combined with improving the development of hypoxemia and reducing the chance of patients turning into severe or even critical type.

The third to seventh editions of the "Diagnosis and Treatment Protocol for Novel Coronavirus Pneumonia (Trial)" specially proposes principles of traditional Chinese medicine treatment and evidence-based treatment protocols according to the clinical manifestations of patients. For the treatment of hypoxemia in novel coronavirus pneumonia, "Diagnosis and Treatment Protocol for Novel Coronavirus Pneumonia (Trial Version 6)" published on February 18, 2020 mentioned for the first time " for patients with progressive deterioration of oxygenation index, rapid progression of imaging, and over-activation of the organism's inflammatory response ......".

Compound Salvia Microdrop Pill (T89) is a traditional Chinese medicine developed by Tasly Ltd. for promoting blood circulation and removing blood stasis, regulating "qi" and relieving pain. It is used for chest tightness and angina pectoris. Its main ingredients are Salvia, Panax notoginseng and borneol. It has clinical effect of treating acute myocardial infarction and acute myocardial ischemia.

T89 is a medicament prepared from natural extracts. It is currently filed for clinical study in the U.S. FDA under the number T89. This product has been used in China for more than 25 years and has a good safety. Due to the invasion of the covid-19 virus, patients with novel coronavirus pneumonia have elevated myocardial enzymes with systemic inflammatory responses, and even causing multiple organ failure and central nervous system damage. The reasons for this are systemic thrombosis, microcirculation disorders, inflammatory factor storm, ROS invasion and hypoxia after virus invasion. These diseases lead to increased mortality, especially in patients with underlying diseases such as cardiovascular disease, hypertension and diabetes. To date, there is no recommended medication for COVID-19 hypoxemia.

### SUMMARY OF THE INVENTION

To solve the above technical problems, the present invention provides an application of a traditional Chinese medicine composition in the preparation of a novel coronavirus pneumonia medicament, the traditional Chinese medicine composition is composed of 50.0%∼99.9% by weight of Salvia miltiorrhiza and Panax notoginseng extract and 0.1%~50.0% by weight of borneol, wherein the Salvia miltiorrhiza and Panax notoginseng extract contains the following components, the weight ratios of each component are:

Danshensu (i.e., salvianic acid A): salvianolic acid T: protocatechuic aldehyde: salvianolic acid D: rosmarinic acid: salvianolic acid B: salvianolic acid A:Notoginsenoside R1: ginsenoside Rg1: ginsenoside Re: ginsenoside Rb1: ginsenoside Rd:Dihydrotanshinone I: tanshinone I: cryptotanshinone: tanshinone IIA = (2∼6): (0.5∼2): (1∼3): (0.2∼1): (0.2∼1): (0.5∼2): (0.5∼2): (0.2∼1): (1∼4): (0.1∼0.5): (1∼4): (0.1∼1): (0.01∼0.05): (0.05∼0.1): (0.02∼0.1): (0.1-0.5).

In patients with Covid-19 pneumonia, due to the invasion of Covid-19 virus, patients have elevated cardiac enzymes accompanied by systemic inflammatory responses, and even cause multiple organ failure and central nervous system damage. The reason of such is The reasons for this are systemic thrombosis, microcirculation disorders, inflammatory factor storm, ROS invasion and hypoxia after virus invasion. The traditional Chinese medicine composition of the present invention can effectively relieve the symptoms of patients with novel coronavirus pneumonia, improve the microcirculation disorder of patients, reduce the formation of pulmonary thrombus, and inhibit disseminated intravascular coagulation (DIC), thereby reduces the mortality rate of patients with moderate to severe novel coronavirus pneumonia.

Further, the traditional Chinese medicine composition of the present invention can prevent or reduce the formation of thrombus in patients with novel coronavirus pneumonia, and reduce the formation of serous fluid, fibrinous exudate and pulmonary hyaline membrane in alveolar space; reduce alveolar septum congestion, edema, reduce mononuclear and lymphocyte infiltration and the formation of intravascular hyaline thrombus; and is capable of inhibiting platelet aggregation and adhesion, thus preventing the formation of systemic thrombosis.

Further, the traditional Chinese medicine composition of the present invention, by regulating vasomotion, can improve the vascular function and improve microcirculation blood perfusion, improve the exchange of oxygen and nutrients in the heart, lung and other organs, thereby improving the microcirculatory disorder in patients with novel coronavirus pneumonia.

Further, the traditional Chinese medicine composition of the present invention can inhibit disseminated intravascular coagulation (DIC) in patients with novel coronavirus pneumonia, and reduce mortality rate.

Further, according to symptoms in clinical classification: mild (mild clinical symptoms, image without pneumonia manifestations); common (fever, respiratory symptoms, and image with pneumonia manifestations); severe (respiratory distress, RR ≥ 30 times/min; in resting state, finger oxygen saturation ≤ 93%; arterial partial pressure of oxygen (PaO2)/inhaled oxygen concentration (FiO2) ≤ 300mmHg) and critically (presence of respiratory failure and need for mechanical ventilation; presence of shock; combined with other organ failure, requires ICU monitoring and treatment). The traditional Chinese medicine composition of the present invention is capable of treating common, moderate and severe pneumonia.

The traditional Chinese medicine composition of the present invention, wherein, the traditional Chinese medicine composition is composed of 75.0%-99.9% of Salvia miltiorrhiza and Panax notoginseng extract and 0.1%-25.0% of borneol by weight percentage.

The traditional Chinese medicine composition of the present invention is composed of 90.0%-99.9% of Salvia miltiorrhiza and Panax notoginseng extract and 0.1%-10.0% of borneol by weight percentage.

The traditional Chinese medicine composition of the present invention, wherein, the Salvia miltiorrhiza and Panax notoginseng extract contains the following components, and the weight ratio of each component is:

Danshensu: salvianolic acid T: protocatechuic aldehyde: salvianolic acid D: rosmarinic acid: salvianolic acid B: salvianolic acid A:Notoginsenoside R1: ginsenoside Rg1: ginsenoside Re: ginsenoside Rb1: ginsenoside Rd:Dihydrotanshinone I: tanshinone I: cryptotanshinone: tanshinone IIA = (3~4):(0.9~1.2): (1.4∼2.0): (0.5∼0.7): (0.5∼0.9): (1∼1.6): (0.7∼1.2): (0.5∼0.9): (1.8∼2.8): (0.2∼0.4): (1.7∼2.2): (0.2∼0.6): (0.03∼0.04): (0.07∼0.08): (0.05∼0.06): (0.26∼0.28).

Further preferred Chinese medicine composition, wherein, said Salvia miltiorrhiza and Panax notoginseng extract contains the following components, and the weight ratio of each component is:

Danshensu: salvianolic acid T: protocatechuic aldehyde: salvianolic acid D: rosmarinic acid: salvianolic acid B: salvianolic acid A: Notoginsenoside R1: ginsenoside Rg1: ginsenoside Re: ginsenoside Rb1: ginsenoside Rd: Dihydrotanshinone I: tanshinone I: cryptotanshinone: tanshinone IIA= 3.6: 1.1: 1.7: 0.6: 0.7: 1.3: 0.9: 0.7: 2.4: 0.3: 1.8: 0.4: 0.03: 0.07: 0.06: 0.27.

In the traditional Chinese medicine composition of the present invention, the Salvia miltiorrhiza and Panax notoginseng extract is prepared from raw materials according to the following parts by weight: 75-90 parts of Salvia miltiorrhiza and 10-25 parts of Panax notoginseng.

In the traditional Chinese medicine composition of the present invention, the Salvia miltiorrhiza and Panax notoginseng extract is prepared from raw materials in the following parts by weight: 82-84 parts of Salvia miltiorrhiza and 16-17 parts of Panax notoginseng.

The traditional Chinese medicine composition of the present invention can be made into various formulations, such as injections, tablets, capsules, dropping pills/micro-dropping pills and micro-dropping capsules, etc., preferably micro-drop pills. The "micro-dropping pill" refers to a drop pill with a smaller volume compared with the existing dropping pill. Specifically, it refers to a drop pill with a particle size of 0.2 mm-4 mm, particularly a drop pill with a particle size of 0.2 mm-2 mm, preferably a particle size of 1 mm-2 mm.

The micro-dropping pill of the present invention is prepared from the traditional Chinese medicine composition and the dropping pill matrix in a weight ratio of 1: 5-5:1.

The preparation method of the micro-dropping pill of the present invention, the method comprises the following steps:
(1) Material homogenizing step: put the raw materials and dropping pill matrix into the homogenizing tank, and mix evenly at 1000∼5000 rpm for 1∼200 min, then homogenize the raw materials at 3000∼10000rpm for 1∼100min. In the homogenizing process, the temperature is maintained at 60 ~ 100°C to obtain a molten medicinal solution, and the weight ratio of the raw material to the dropping pill matrix is 1:5 ~ 5:1;
(2) Dripping step: transfer the above molten medicinal solution to the dripper, under the conditions of dripper temperature at 70~300°C, dripping vibration frequency at 2∼2000 Hz, dripping pressure at 0.5∼4.0 Bar, and acceleration at 1∼20 G, micro-dropping pills are made by vibration of the dripper, and the dripping speed matches the speed of the material homogenizing in Step (1);
(3) Condensation step: the droplets are rapidly cooled in a cooling gas, and solidified into solid dropping pills with a particle size of 0.2 mm~4.0 mm, and the temperature of the cooling gas is below 0°C.

In the above Step (1), the dropping pill matrix includes one or more combinations of PEGs, sorbitol, xylitol, lactitol, maltose, starch, methyl cellulose, sodium carboxymethyl cellulose, hydroxypropyl methyl cellulose, Arabic gum, alginic acid, dextrin, cyclodextrin, agar, lactose; the preferred dropping pill matrix is solid PEG, such as PEG-1000, PEG-2000, PEG-3000, PEG-4000 , PEG-5000, PEG-6000, PEG-7000, and PEG-8000, more preferably one or more combinations of PEG-1000, PEG-2000, PEG-3000, PEG-4000, PEG-6000, and PEG-8000, most preferably PEG-6000, PEG-4000 or a combination of PEG-4000 and PEG-6000.

Further preferred preparation method, wherein, the method comprises the following steps:
(1) Material homogenizing step: put the raw material and dropping pill matrix into the homogenizing tank, and mix evenly at 1000~5000rpm, and then homogenize the material at 3000~10000rpm for 20∼80 min. During the homogenizing process, keep the temperature at 80 ~ 100°C to obtain a molten medicinal solution, and the weight ratio of raw materials to the dropping pill matrix is 1:3 ~ 3:1;
(2) Dripping step: transfer the above molten medicinal solution to the dripper, under the conditions of dripper temperature at 70~200°C, dripping vibration frequency at 20∼300 Hz, dripping pressure at 0.5∼4.0 Bar, and acceleration at 1~15 G, micro-dropping pills are made by vibration of the dripper, and the dripping speed matches the speed of the material homogenizing in Step (1);
(3) Condensation step: the droplets are rapidly cooled in a cooling air, and solidified into solid dropping pills with a particle size of 0.2 mm~4.0 mm, and the temperature of the cooling air is below 0°C.

In the above Step (1), the weight ratio of the raw material and the dropping pill matrix is 1:3∼3:1, evenly mixed at 3000∼5000 rpm for 10∼60 min, and then homogenized at 4000~9000rpm for 5 ~ 30min, during the homogenizing process, keep the temperature at 70 ∼ 90°C; Preferably, the weight ratio of raw materials and the dropping pill matrix is 1:(1 ~ 3), evenly mixed at 3000 ~ 4000rpm for 10∼30 min, and then, the materials are homogenized at 4000~6000rpm for 6~30min, during the homogenizing process, keep the temperature at 75~85°C.

In the above Step (2), the temperature of the dripper is 70∼100°C, preferably 75~85°C; the dripping vibration frequency is 50∼300 Hz, preferably 100∼200 Hz, more preferably 90∼200 Hz, more preferably 130∼140 Hz, most preferably 137 Hz; acceleration is 3.5~4.5G, preferably 4.0G; dripping pressure is 1.0∼3.0 Bar, preferably 1.8 Bar; dripping speed is 10∼40 kg/h, preferably 12∼30 kg/h, further preferably 15∼25 kg/h.

In the above Step (3), the gas is air, nitrogen and inert gas; the cooling temperature is 0∼-150°C, preferably -60∼-140°C, more preferably -80∼-120°C; the diameter of the dropping pill is 1.0mm~2.0mm.

The preparation method of the present invention also comprises a drying step as Step (4), in which the pills are dried by fluidized drying equipment at -20∼100°C, preferably - 20~90°C for 1∼4 hours, to obtain plain pellets.

Further preferably, Step (4) adopts a gradient heating drying method: forming a fluidized state at -20~30°C, drying at 15~35°C for 10~120min, drying at 35~55°C for 10~60min, and drying at 55∼100°C °C for 0~60min; preferably, the gradient heating drying method is carried out as follows: forming a fluidized state at 0~20°C, drying at 25°C for 60min, drying at 45°C for 30min, and drying at 55°C for 0~30min.

The preparation method of the present invention also comprises a coating step as Step (5), in which the plain pellets obtained in Step (4) in a fluidized state are coated at a temperature of 30-65°C; the coating liquid concentration is 5~25wt%, preferably 18∼20wt%, wherein, the coating material is selected from: shellac, cellulose acetate phthalate, methyl acrylate, methyl methacrylate or Opadry; the weight ratio of the coating material to the plain pellets is 1:50∼1:10, preferably 1:50∼1:25.

Before Step (1), the preparation method of the present invention may further comprise a material premixing step, after adding water to the medicinal extract or powder, stirring at 30-80° C for more than 10 min to obtain a medicinal premixture.

For better proving the beneficial effects of the present invention, the following examples are used for illustration.

### DETAILED DESCRIPTION OF THE INVENTION

In one embodiment, the present invention provides a traditional Chinese medicine composition. The traditional Chinese medicine composition is composed of 50.0%-99.9% of Salvia miltiorrhiza and Panax notoginseng extract and 0.1%-50.0% of borneol by weight percentage, wherein the Salvia miltiorrhiza and Panax notoginseng extract contains (parts by weight): Danshensu: salvianolic acid T: protocatechuic aldehyde: salvianolic acid D: rosmarinic acid: salvianolic acid B: salvianolic acid A: notoginsenoside R1: ginsenoside Rg1: ginsenoside Re: ginsenoside Rb1: ginsenoside Rd: dihydrotanshinone I: tanshinone I: cryptotanshinone: tanshinone IIA = (2∼6): (0.5∼2): (1∼3): (0.2∼1): (0.2∼1) : (0.5∼2): (0.5∼2): (0.2∼1): (1∼4): (0.1∼0.5): (1∼4): (0.1∼1): (0.01∼0.05): (0.05∼0.1): (0.02∼0.1): (0.1∼0.5).

Preferred traditional Chinese medicine composition of the present invention is composed of 75.0% ~ 99.9% of Salvia miltiorrhiza and Panax notoginseng extract, and 0.1% ~ 25.0% of borneol by weight percentage.

A further preferred traditional Chinese medicine composition of the present invention is composed of 90.0% ~ 99.9% of Salvia miltiorrhiza and Panax notoginseng extract, and 0.1% ~ 10.0% of borneol by weight percentage.

In the aforementioned traditional Chinese medicine composition, the Salvia miltiorrhiza and Panax notoginseng extract preferably contains (weight portion):

Danshensu: salvianolic acid T: protocatechuic aldehyde: salvianolic acid D: rosmarinic acid: salvianolic acid B: salvianolic acid A: notoginsenoside R1: ginsenoside Rg1: ginsenoside Re: ginsenoside Rb1: ginsenoside Rd: dihydrotanshinone I: tanshinone I: cryptotanshinone: tanshinone IIA=(3∼4): (0.9∼1.2): (1.4∼2.0): (0.5∼0.7): (0.5∼0.9) : (1∼1.6): (0.7∼1.2): (0.5∼0.9): (1.8∼2.8): (0.2∼0.4): (1.7∼2.2): (0.2∼0.6): (0.03∼0.04): (0.07∼0.08): (0.05∼0.06): (0.26∼0.28).

In aforementioned traditional Chinese medicine composition, the Salvia miltiorrhiza and Panax notoginseng extract more preferably contains (weight portion):
Danshensu: salvianolic acid T: protocatechuic aldehyde: salvianolic acid D: rosmarinic acid: salvianolic acid B: salvianolic acid A: notoginsenoside R1: ginsenoside Rg1: ginsenoside Re: ginsenoside Rb1: ginsenoside Rd: dihydrotanshinone I: tanshinone I: cryptotanshinone: tanshinone IIA = 3.6: 1.1: 1.7: 0.6: 0.7: 1.3: 0.9: 0.7: 2.4: 0.3: 1.8: 0.4: 0.03: 0.07: 0.06: 0.27.

The aforementioned traditional Chinese medicine composition can be prepared by extracting and processing Salvia miltiorrhiza and Panax notoginseng to obtain the extract and then adding borneol for mixing.

The traditional Chinese medicine composition of the present invention is preferably prepared by the following method:
(1) Raw materials of Salvia miltiorrhiza and Panax notoginseng are decocted with water under alkaline conditions, the decoction is filtered, the filtrate is concentrated and alcohol-precipitated, the supernatant is filtered, and the ethanol is recovered to obtain the extract (or the extract is further dried), i.e. Salvia miltiorrhiza and Panax notoginseng extract;
(2) Add borneol into the Salvia miltiorrhiza and Panax notoginseng extract and mix them evenly,

Wherein, Salvia miltiorrhiza and Panax notoginseng can be decocted respectively in the water under the same alkaline condition; or decocted in the water under the same alkaline condition together.

Preferably, the Salvia miltiorrhiza and Panax notoginseng extract can be prepared by the following method:
Step (1): Decoct Salvia miltiorrhiza and Panax notoginseng for 1 ~ 3 times in aqueous solution under alkaline condition, each time for 1 ~ 3 hours, filter to obtain Filtrate 1;
Step (2): Add water into the dregs of decoction for decocting 1 ~ 3 times, each time for 1 ~ 3 hours, filter to obtain Filtrate II;
Step (3): Filtrate I and Filtrate II are combined and concentrated, the concentrated solution is alcohol-precipitated and left to stand, the supernatant is filtered, and the ethanol is recovered to obtain the concentrated extract, or dry the extract to obtain the Salvia miltiorrhiza and Panax notoginseng extract.

Wherein, the alkaline aqueous solution in Step (1) comprises, but is not limited to one or more aqueous solutions of sodium bicarbonate, sodium carbonate, sodium hydrogen phosphate, sodium dihydrogen phosphate, sodium hydroxide, potassium hydroxide, magnesium hydroxide, with a pH value of 7.5 ~ 9.0, the added quantity of alkali in the aforementioned alkaline aqueous solution is 1 ~ 4.5 weight % of that of the raw materials, preferably 2.25 ~ 3 weight %, to ensure that Danshensu sodium and salvianolic acid T are extracted thoroughly .

In Step (3), preferably add 50% ~ 100% ethanol, preferably 95% ethanol and carry out alcohol precipitation, and preferably alcohol-precipitate to the concentration of 60% ~ 75% ethanol.

More preferably, Salvia miltiorrhiza and Panax notoginseng extract of the present invention is prepared by the following method:
Step (1): Cut the raw materials of Salvia miltiorrhiza into pieces of less than 5 cm long, preferably 1-2 cm, pulverize the raw materials of Panax notoginseng into granules of 1 cm, and weigh 2.25 ~ 3% by weight of sodium bicarbonate accounting for the total weight of the raw materials. Put the weighed raw materials of Salvia miltiorrhiza, Panax notoginseng and sodium bicarbonate into the extracting tank, add 5 times the amount of process water to each tank, heat to boil and keep boiling for 2h±20min, and filter the mixture,
Step (2): Extract the dregs of decoction for the second time, adds 4 times the amount of water, heat to boil and keep boiling for 1h ± 15min, filter the mixture, and discard the dregs of decoction;
Step (3): Extract solution is concentrated under reduced pressure to a relative density of 1.16 ∼ 1.20 (80 ± 5 °C) or the sugar content of corresponding 48% ~ 52% to obtain a concentrated solution, and the concentrated solution is pushed into alcohol precipitation tank, add an appropriate amount of ethanol to adjust to an alcohol content of 65% ~ 70%, and allow it to stand for 12 ~ 24 hours until a complete precipitation status, separate the supernatant and discard the precipitation, and the supernatant is concentrated to obtain an extract, or dry the extract, to obtain the Salvia miltiorrhiza and Panax notoginseng extract.

Wherein, the 5 times amount in Step (1) refers to the 5 times amount of the total weight of the raw materials, similarly, the 4 times amount in Step (2) refers to the 4 times amount of the total weight of the dregs of decoction.

The traditional Chinese medicine composition in the present invention is prepared from the following raw materials by weight: 75 ~ 90 parts of Salvia miltiorrhiza, 10 ~ 25 parts of Panax notoginseng and 0.1 ~ 4 parts of borneol.

A preferred traditional Chinese medicine composition is prepared from the following raw materials by weight: 80 ~ 86 parts of Salvia miltiorrhiza, 15 ~ 18 parts of Panax notoginseng and 0.2 ~ 2 parts of borneol.

The most preferred traditional Chinese medicine composition is prepared from the following raw materials by weight: 82 ~ 84 parts of Salvia miltiorrhiza, 16 ~ 17 parts of Panax notoginseng and 0.4 ~ 1.2 parts of borneol.

The traditional Chinese medicine composition of the present invention can be either an extract or a powder.

In one embodiment, the present invention provides a pharmaceutical formulation of the traditional Chinese medicine composition, and the pharmaceutical formulation comprises the traditional Chinese medicine composition of the present invention and one or more pharmaceutically acceptable carriers. The traditional Chinese medicine composition of the present invention may account for 0.1% ~ 99.9% by weight in its formulation, with the remainder being pharmaceutically acceptable carriers.

The pharmaceutical formulation of the present invention is the form of unit dose pharmaceutical formulation, and the unit dose refers to the unit of the formulation, such as per tablet for tablets, per capsule for capsules, per bottle for oral liquids, per sachet for granules, etc., and can be prepared by any method known in the pharmaceutical art. All the methods include the step of combining the traditional Chinese medicine composition of the present invention with a carrier that constitutes one or more auxiliary components. Generally speaking, the preparation process of the formulation is as follows: the traditional Chinese medicine composition of the present invention is evenly and tightly combined with a liquid carrier, or a finely pulverized solid carrier, or a combination thereof, and then, if necessary, the product is shaped into a required formulation. The traditional Chinese medicine composition of the present invention and a pharmaceutically acceptable carrier can generally be used to prepare the pharmaceutical formulation of the present invention using standard pharmaceutical techniques, including mixing, granulation and compression. It is well known to those skilled in the art that the forms and characters of the pharmaceutically acceptable carrier or diluent depend on the amount of active ingredient with which it is mixed, the route of administration and other known aspects.

Its form of the pharmaceutical formulation can be in any pharmaceutically acceptable dosage form, including: tablets, sugar-coated tablets, film-coated tablets, enteric-coated tablets, capsules, hard capsules, soft capsules, oral liquids, buccal preparations, granules, electuaries, pills, powders, ointments, pellets, suspensions, powder flux, solutions, injections, suppositories, emulsions, soft ointments, plasters, creams, sprays, drops, patches. The formulation of the present invention is preferably in an oral dosage form, such as capsules, tablets, oral liquids, granules, pills, powders, pellets, ointments, etc.

The formulation for oral administration may comprise commonly used excipients such as binders, fillers, diluents, tableting agents, lubricants, disintegrating agents, coloring agents, flavoring agents and wetting agents, tablets may be coated if necessary.

Suitable fillers include cellulose, mannitol, lactose and other similar fillers. Suitable disintegrating agents include starch, polyvinylpyrrolidone and starch derivatives such as sodium starch glycolate. Suitable lubricants include, for example, magnesium stearate. Suitable pharmaceutically acceptable wetting agents include sodium dodecyl sulfate.

Solid oral compositions can be prepared by conventional methods such as mixing, filling, tableting, etc. Repeated mixing allows the active substance to be distributed throughout those compositions where large amounts of fillers are used.

Oral liquid formulations can be in the form of, for example, aqueous or oily suspensions, solutions, emulsions, syrups or elixirs, or alternatively, may be a dry product which may be compounded with water or other suitable carrier prior to use. Such liquid formulations may contain conventional additives such as suspending agents, for example sorbitol, syrup, methylcellulose, gelatin, hydroxyethylcellulose, carboxymethylcellulose, aluminum stearate gel or hydrogenated edible fats; emulsifiers, such as lecithin, dehydrated sorbitol monooleic acid, or Arabic gum; non-aqueous carriers (which may include edible oils), such as almond oil, fractionated coconut oil, oily esters such as glycerol esters, propylene glycol, or ethanol; preservatives agents such as methylparaben or propylparaben or sorbic acid; and if necessary, it may contain conventional flavoring agents or coloring agents.

For injections, the prepared liquid unit dosage form contains the active substance of the present invention and a sterile carrier. Depending on the carrier and the concentration, the compound can be suspended or dissolved. Solutions are usually prepared by dissolving the active substance in a carrier, being filter sterilized before filling into a suitable vial or ampoule, and sealed. Excipients such as local anesthetics, preservative agents and buffering agents can be also dissolved in such carrier. To improve the stability, the composition can be frozen after filling into a vial with the water removed under vacuum condition.

A suitable pharmaceutically acceptable carrier may be selectively added during the preparation of the medicament, and the pharmaceutically acceptable carrier is selected from: mannitol; sorbitol; sodium metabisulfite; sodium bisulfite; sodium thiosulfate; cysteine hydrochloride; thioglycolic acid; methionine; vitamin C; disodium EDTA; calcium sodium EDTA; carbonate, acetate, phosphate of monovalent alkali metals or the aqueous solution thereof; hydrochloric acid; acetic acid; sulfuric acid; phosphoric acid; amino acid; sodium chloride; potassium chloride; sodium lactate; xylitol; maltose; glucose; fructose; dextran; glycine; starch; sucrose; lactose; mannitol; silicon derivatives; cellulose and derivatives thereof; alginate; gelatin; polyvinylpyrrolidone; glycerin; Tween 80; agar; calcium carbonate; calcium bicarbonate; surfactant; polyethylene glycol; cyclodextrin; β-cyclodextrin; phospholipid materials; kaolin; talc; calcium stearate; magnesium stearate, etc.

In the present invention, the traditional Chinese medicine composition is preferably prepared into a dropping pill formulation, and more preferably a micro-dropping pill formulation.

In another embodiment, the present invention provides a compound Salvia miltiorrhiza micro-dropping pill, and the compound Salvia miltiorrhiza micro-dropping pill is made of the traditional Chinese medicine composition and the dropping pill matrix of a weight ratio of 1:5 ~ 5:1. Preferably, the compound Salvia miltiorrhiza micro-dropping pill of the present invention is made of the traditional Chinese medicine composition and the dropping pill matrix of a weight ratio of 1:3 ~ 3:1; most preferably, the compound Salvia miltiorrhiza micro-dropping pill of the present invention is made of the traditional Chinese medicine composition and a dropping pill matrix of a weight ratio of 1:(1~3).

The preparation method of compound Salvia miltiorrhiza micro-dropping pill of the present invention comprises the following steps:
(1) Material homogenizing step: put the raw materials and dropping pill matrix into the homogenizing tank, and mix evenly at 1000∼5000 rpm for 1∼200 min, then homogenize the raw materials at 3000∼10000rpm for 1∼100min. In the homogenizing process, the temperature is maintained at 60 ~ 100°C to obtain a molten medicinal solution, and the weight ratio of the raw material to the dropping pill matrix is 1:5 ~ 5:1;
(2) Dripping step: transfer the above molten medicinal solution to the dripper, under the conditions of dripper temperature at 70∼300°C, dripping vibration frequency at 2∼2000 Hz, dripping pressure at 0.5∼4.0 Bar, and acceleration at 1∼20 G, micro-dropping pills are made by vibration of the dripper, and the dripping speed matches the speed of the material homogenizing in Step (1);
(3) Condensation step: the droplets are rapidly cooled in a cooling gas, and solidified into solid dropping pills with a particle size of 0.2 mm~4.0 mm, and the temperature of the cooling gas is below 0°C.

Preferably, the preparation method of compound Salvia miltiorrhiza micro-dropping pill of the present invention comprises the following steps:
(1) Material homogenizing step: put the raw materials and dropping pill matrix into the homogenizing tank, and mix evenly at 1000∼5000 rpm for 1∼200 min, then homogenize the raw materials at 3000∼10000rpm for 1∼100min. In the homogenizing process, the temperature is maintained at 80 ~ 100°C to obtain a molten medicinal solution, and the weight ratio of the raw material to the dropping pill matrix is 1:3 - 3:1;
(2) Dripping step: transfer the above molten medicinal solution to the dripper, under the conditions of dripper temperature at 70~200°C, dripping vibration frequency at 2∼300 Hz, dripping pressure at 0.5∼4.0 Bar, and acceleration at 1~15G, micro-dropping pills are made by vibration of the dripper, and the dripping speed matches the speed of the material homogenizing in Step (1);
(3) Condensation step: the droplets are rapidly cooled in a cooling gas, and solidified into solid dropping pills with a particle size of 0.2 mm~4.0 mm, and the temperature of the cooling gas is below 0°C.

Wherein, in the aforementioned Step (1), the dropping pill matrix includes one or more combinations of PEGs, sorbitol, xylitol, lactitol, maltose, starch, methyl cellulose, sodium carboxymethyl cellulose, hydroxypropyl methyl cellulose, Arabic gum, alginic acid, dextrin, cyclodextrin, agar, lactose; the preferred dropping pill matrix is solid PEG, such as PEG-1000, PEG-2000, PEG-3000, PEG-4000 , PEG-5000, PEG-6000, PEG-7000, and PEG-8000, more preferably one or more combinations of PEG-1000, PEG-2000, PEG-3000, PEG-4000, PEG-6000, and PEG-8000 , most preferably PEG-6000, PEG-4000 or a combination of PEG-4000 and PEG-6000. The homogenization in Step (1) is capable of improving the content uniformity from the previous RSD=10% to 7%.

Preferably, in aforementioned Step (1), the weight ratio of the raw materials and the dropping pill matrix is 1:3 ~ 3:1, and they are homogenized at 3000 ~ 5000rpm for 10 ~ 60min, then, then homogenize the raw materials at 4000 ~ 9000rpm for 5 ~ 30min. In the homogenizing process, the temperature is maintained at 70 ~ 90°C; most preferably, the weight ratio of the raw materials and the dropping pill matrix is 1:(1 ~ 3), and they are homogenized at 3000 ~ 4000rpm for 10 ~ 30min, then, homogenize the raw materials at 4000 ~ 6000rpm for 6 ~ 30min. In the homogenizing process, the temperature is maintained at 75 ~ 85°C.

Wherein, preferably, in aforementioned Step (2), the dripper temperature is 70 ~ 100°C, preferably 75 ~ 85°C; the vibration frequency of dripping is 50 ~ 300 Hz, preferably 100 ~ 200 Hz, more preferably 90 ~ 200 Hz, more preferably 130 ~ 140 Hz, most preferably 137 Hz; the acceleration is 3.5 ∼ 4.5 G, preferably 4.0 G; the dripping pressure is 1.0 ~ 3.0 Bar, preferably 1.8 Bar; the dripping speed is 10 ~ 40 kg/h, preferably 12 ~30 kg/h, more preferably 15∼25 kg/h.

In aforementioned Step (3), gas cooling refers to utilizing cryogenic cold trap to rapidly cool the falling droplets to solidify them. The temperature range of the cooling gas is 0°C or lower, preferably 0 ∼ -150°C, preferably -60°C ∼ -140°C, more preferably -80°C ~ -120°C, and preferably the cooling gas is air, nitrogen, or inert gas. The particle size of the obtained micro-dropping pill is preferably 1.0 mm ~ 2.0 mm.

Further, the preparation method of the micro-dropping pill of the present invention also comprises a drying step as Step (4), in which the pills are dried by fluidized drying equipment at -20~100°C, preferably -20~90°C for 1~4 hours, to obtain plain pellets. Specifically, the low-temperature dropping pills after completion of dropping in Step (3) are dried in a fluidized bed at a temperature of 40 ~ 150°C, preferably 40 ~ 60°C, with a drying time of 1 ~ 4 hours, preferably 1 ~ 3 hours, and most preferably 2h, to obtain plain pellets.

In aforementioned Step (4), preferably a gradient heating drying method is used: forming a fluidized state at -20~30°C, drying at 15~35°C for 10~120min, drying at 35~55°C for 10~60min, and drying at 55~100°C °C for 0~60min; preferably, the gradient heating drying method is carried out as follows: forming a fluidized state at 0~20°C, drying at 25°C for 60min, drying at 45°C for 30min, and drying at 55°C for 0~30min. This step keeps the dropping pills in the fluidized state, solves the problem of sticking of the dropping pills, and also improves the efficiency to a capacity of 30kg/h.

The preparation method of the present invention also comprises a coating step as Step (5), in which the plain pellets obtained in Step (4) in a fluidized state are coated at a temperature of 30~65°C; the coating liquid concentration is 5~25wt%, preferably 18~20wt%, wherein, the coating material is selected from: shellac, cellulose acetate phthalate, methyl acrylate, methyl methacrylate or Opadry; the weight ratio of the coating material to the plain pellets is 1:50∼1:10, preferably 1:50∼1:25.

To better implement the preparation method of the micro-dropping pill of the present invention, preferably before Step (1), the preparation method of the present invention may further comprise a material premixing step, after adding water to the medicinal extract or powder, stirring at 30-80° C for more than 10 min to obtain a medicinal premixture and keep moisture homogeneity. This step can compensate the disadvantages of dry powder feeding..

The dropping pills obtained by the method of the present invention can be directly packaged, and can also be packed into capsule shells to produce capsules. After being made into capsule formulations, a capsule-by-capsule weighing step may be added, and the filled capsules are weighed capsule-by-capsule at high speed prior to packaging to remove any possible unqualified capsules.

### Examples

The process of the present invention is further described in detail below by way of embodiments. The embodiments are used only to illustrate the present invention and are not intended to constitute a limitation of the invention.

The detection method of each component of Salvia miltiorrhiza and Panax notoginseng extract

In the following embodiments, the content of each component of each traditional Chinese medicine composition: Danshensu, salvianolic acid T, protocatechuic aldehyde, salvianolic acid D, rosmarinic acid, salvianolic acid B, salvianolic acid A, dihydrotanshinone I, tanshinone I, cryptotanshinone, tanshinone IIA, notoginsenoside R1, ginsenoside Rg1, ginsenoside Re, ginsenoside Rb1, ginsenoside Rd were determined according to the following method.

The detection of phenolic acids and tanshinones components:
The preparation of solutions of the reference substances and the sample
The reference substance solutions: a certain amount of reference substances ofDanshensu, salvianolic acid T, protocatechuic aldehyde, salvianolic acid D, rosmarinic acid, salvianolic acid B, salvianolic acid A, dihydrotanshinone I. tanshinone I, cryptotanshinone, and tanshinone IIA were precisely weighed in a 10ml volumetric flask respectively, and dissolved in methanol and diluted to a certain graduation line, and diluted and shaken as needed to prepare solutions of the following concentrations: 0.0315mg/ml Danshensu, 0.04596mg/ml salvianolic acid T, 0.07556mg/ml protocatechuic aldehyde, 0.04385mg/ml salvianolic acid D, 0.04263mg/ml rosmarinic acid, 0.04248mg/ml salvianolic acid B, 0.1118mg/ ml salvianolic acid A, 0.02098mg/ml dihydrotanshinone I, 0.02085mg/ml tanshinone I, 0.02442mg/ml cryptotanshinone, 0.01992mg/ml tanshinone IIA, then filtered through a 0.22 µm membrane to obtain the reference solutions.

The sample solution: 0.1g Salvia miltiorrhiza and Panax notoginseng extract sample was precisely weighed in a 10ml volumetric flask, and dissolved in pure water, and diluted to a certain graduation line, then filtered through a 0.22 µm membrane to obtain the sample solution.

Detection method: each 10 µl of the reference substance solutions and the sample solution were precisely drawn respectively, and injected into ultra-high performance liquid chromatograph to run the detection.
Chromatographic column: Agilent Zorbax SB C18 (4.6 × 250mm, 5 µm)
Flow rate: 0.5 mL/min
Column temperature: 30 °C

Detection wavelength: 281nm, and the elution conditions are as follows in Table 1:

**Table 1**

| Time (min) | A (%) | B (%) |
|---|---|---|
| | Water (0.02% methanoic acid) | acetonitrile (0.02% methanoic acid) |
| 0 | 90 | 10 |
| 15 | 80 | 20 |
| 25 | 75 | 25 |
| 30 | 74 | 26 |
| 45 | 54 | 46 |
| 50 | 48 | 52 |
| 62 | 28 | 72 |
| 70 | 0 | 100 |
| 76 | 0 | 100 |

The detection of the saponins component:
The preparation of reference substance solution: Appropriate amount of notoginsenoside R1 reference substance, ginsenoside Rg1 reference substance, ginsenoside Rb1 reference substance, ginsenoside Re reference substance, ginsenoside Rd reference substance were precisely weighed and made into solutions containing 0.5mg, 2.0mg, 1.0mg, 0.5mg, 0.5mg, 0.5mg and 1.0mg per 1ml respectively by adding methanol.

The preparation of sample solution: 0.1g sample was precisely weighed and dissolved in 10ml of 4% ammonia solution, and passed through D101 type macroporous adsorption resin column (internal diameter is 0.7cm, column height is 5cm), and washed with 30ml of water, then eluted with 30ml of 30% methanol, and eluted with 10ml of methanol. The methanol solution was collected into a 10ml volumetric flask, and was shaken evenly.

Chromatographic condition and system suitability test: take the silica gel bonded with octadecyl silane was used as filler; acetonitrile was used as mobile phase A, water was used as mobile phase B, a gradient elution was carried out according to Table 2 below; the flow velocity was 1.0ml per minute; the detection wavelength was 203nm; the column temperature was 30°C; the recording time was 75 min.

**Table 2 Reference table for mobile phase gradient elution**

| Time (min) | **mobile phase** A | **mobile phase B** |
|---|---|---|
| 0 | 20 | 80 |
| 25 | 25 | 75 |
| 60 | 40 | 60 |
| 70 | 70 | 30 |
| 75 | 20 | 80 |
| 80 | 20 | 80 |

Detection: Determination: 10 µl of each of the control solution and the sample solution were precisely aspirated and injected into the liquid chromatograph..

The preparation of the traditional Chinese medicine composition of the present invention

### Example 1

820g of the raw material of Salvia miltiorrhiza was cut into pieces of less than 5 cm long, preferably 1 ~ 2 cm, 160g of the raw material of Panax notoginseng was pulverized into granules of 1 cm, and 2.25% of the total weight of the raw materials of sodium bicarbonate was weighed. The weighed raw materials of Salvia miltiorrhiza, Panax notoginseng and sodium bicarbonate were put into the extracting tank, 5 times the amount of process water was added to each tank, and heated to boil and kept boiling for about 2h, and the mixture was filtered, the dregs of decoction were extracted for the second time by adding 5 times the amount of water and heated to boil and was kept boiling for 1h, the mixture was filtered and the dregs of decoction were discarded; the extract was concentrated under reduced pressure to a relative density of 1.16 ∼ 1.20 (80±5°C) or 48 ~ 52% of the corresponding sugar degree to obtain the concentrated solution; the concentrated solution was introduced into the alcohol precipitation tank, and the alcohol content was adjusted to 65%-70% by adding an appropriate amount of ethanol, the mixture was left to stand for 12 hours until a complete precipitation status, the supernatant was separated by discarding the precipitate; the supernatant was concentrated to obtain the extract, which was dried to obtain Salvia miltiorrhiza extract.

The Salvia miltiorrhiza and Panax notoginseng extract was measured by aforementioned detection method, wherein, the Salvia miltiorrhiza and Panax notoginseng extract contains 36mg/g of Danshensu, 11mg/g of salvianolic acid T, 17mg/g of protocatechuic aldehyde, 6mg/g of salvianolic acid D, 7mg/g of rosmarinic acid, 13mg/g of salvianolic acid B, 9mg/g of salvianolic acid A, 17mg/g of notoginsenoside R, 24mg/g of ginsenoside Rg1, 3mg/g of ginsenoside Re, 18mg/g of ginsenoside Rb1, 4mg/g of ginsenoside Rd, 0.3mg/g of dihydrotanshinone I, 0.7mg/g of tanshinone I, 0.6mg/g of cryptotanshinone, and 2.7mg/g of tanshinone IIA.

90g Salvia miltiorrhiza and Panax notoginseng extract and 9g borneol were mixed to obtain the traditional Chinese medicine composition.

### Example 2

75g of the Salvia miltiorrhiza and Panax notoginseng extract prepared in Example 1 and 25g of borneol were mixed evenly to obtain the traditional Chinese medicine composition.

### Example 3

800.0g Salvia miltiorrhiza and 150.0g Panax notoginseng were decocted with water under alkaline condition (pH=9) for 3 times, each time 1 hour, and the decoction was filtered to obtains Filtrate 1; the dregs of decoction was decocted in water for 3 times, each time1 hour, the decoction was filtered to obtain Filtrate II; Filtrate I and Filtrate II were combined and concentrated, the concentrated solution was added with ethanol to an alcohol concentration of 70%, and was left to stand, the supernatant was taken to be filtered, and ethanol was recovered, then concentrated and dried to obtain Salvia miltiorrhiza and Panax notoginseng extract.

The Salvia miltiorrhiza and Panax notoginseng extract is measured by aforementioned detection method, wherein, the Salvia miltiorrhiza and Panax notoginseng extract comprises 40mg/g of Danshensu, 12mg/g of salvianolic acid T, 20mg/g of protocatechuic aldehyde, 7mg/g of salvianolic acid D, 9mg/g of rosmarinic acid, 16mg/g of salvianolic acid B, 12mg/g of salvianolic acid A, 9mg/g of notoginsenoside R1, 28mg/g of ginsenoside Rg1, 4mg/g of ginsenoside Re, 22mg/g of ginsenoside Rb1, 6mg/g of ginsenoside Rd, 0.4mg/g of dihydrotanshinone I, 0.8mg/g of tanshinone I, 0.6mg/g of cryptotanshinone, and 2.8mg/g of tanshinone IIA.

99.9g Salvia miltiorrhiza and Panax notoginseng extract and 0.1g borneol were mixed to obtain the traditional Chinese medicine composition.

### Example 4

90g of the Salvia miltiorrhiza and Panax notoginseng extract prepared in Example 3 and 10g of borneol were mixed evenly to obtain the traditional Chinese medicine composition.

### Example 5

750g Salvia miltiorrhiza and 250g Panax notoginseng were decocted with water under alkaline condition (pH=7.5) for 2 times, each time 2 hours, and the decoction was filtered to obtains Filtrate 1; the dregs of decoction was decocted in water for 2 times, each time 2 hours, the decoction was filtered to obtain Filtrate II; Filtrate I and Filtrate II were combined and concentrated, the concentrated solution was added with ethanol to an alcohol concentration of 70%, and was left to stand, the supernatant was taken to be filtered, and ethanol was recovered, then concentrated and dried to obtain Salvia miltiorrhiza and Panax notoginseng extract.

The Salvia miltiorrhiza and Panax notoginseng extract was measured by aforementioned detection method, wherein, Danshensu, salvianolic acid T, protocatechuic aldehyde, salvianolic acid D, rosmarinic acid, salvianolic acid B, salvianolic acid A, notoginsenoside R1, ginsenoside Rg1, ginsenoside Re, ginsenoside Rb1, ginsenoside Rd, dihydrotanshinone I, tanshinone I, cryptotanshinone, and tanshinone IIA that were contained in the Salvia miltiorrhiza and Panax notoginseng extract were 30 mg/g, 9mg/g, 14mg/g, 5mg/g, 5mg/g, 10mg/g, 7mg/g, 5mg/g, 18mg/g, 2mg/g, 17mg/g, 2mg/g, 0.3mg/g, 0.7 mg/g, 0.5 mg/g, and 2.6 mg/g, respectively.

50g of the Salvia miltiorrhiza and Panax notoginseng extract prepared in Example 1 and 50g of borneol were mixed evenly to obtain the traditional Chinese medicine composition.

### Example 6

99g of the Salvia miltiorrhiza and Panax notoginseng extract prepared in Example 5 and 1g of borneol were mixed evenly to obtain the traditional Chinese medicine composition.

### Example 7

83 parts of Salvia miltiorrhiza and 17 parts of Panax notoginseng were decocted with water under alkaline condition (pH=7.5) for 2 times, each time 2 hours, and the decoction was filtered to obtains Filtrate 1; the dregs of decoction was decocted in water for 2 times, each time 2 hours, the decoction was filtered to obtain Filtrate II; Filtrate I and Filtrate II were combined and concentrated, the concentrated solution was added with ethanol to an alcohol concentration of 70%, and was left to stand, the supernatant was taken to be filtered, and ethanol was recovered, then concentrated and dried to obtain Salvia miltiorrhiza and Panax notoginseng extract; then 1 part of borneol was added and mixed evenly to obtain the traditional Chinese medicine composition. The borneol was available from the market.

The Salvia miltiorrhiza and Panax notoginseng extract was measured by aforementioned detection method, wherein, Danshensu, salvianolic acid T, protocatechuic aldehyde, salvianolic acid D, rosmarinic acid, salvianolic acid B, salvianolic acid A, notoginsenoside R1, ginsenoside Rg1, ginsenoside Re, ginsenoside Rb1, ginsenoside Rd, dihydrotanshinone I, tanshinone I, cryptotanshinone, and tanshinone IIA that were contained in the Salvia miltiorrhiza and Panax notoginseng extract were 40mg/g, 12mg/g, 20mg, 7mg/g, 9mg/g, 16mg/g, 12mg/g, 9mg/g, 28mg/g, 4mg/g, 22mg/g, 6mg/g, 0.4mg/g, 0.8mg/g, 0.6 mg/g, and 2.8 mg/g, respectively.

### Example 8

400g of the raw material of Salvia miltiorrhiza was cut into pieces of 1 ~ 2 cm long, 80g of the raw material of Panax notoginseng was pulverized into granules of 1 cm, and 3% of the total weight of the raw materials of sodium bicarbonate was weighed. The weighed raw materials of Salvia miltiorrhiza, Panax notoginseng and sodium bicarbonate were put into the extracting tank, 5 times the amount of process water was added to each tank, and heated to boil and kept boiling for about 2h±20min, and the mixture was filtered, the dregs of decoction were extracted for the second time by adding 4 times the amount of water and heated to boil and was kept boiling for 1h±15min, the mixture was filtered and the dregs of decoction were discarded; the extract was concentrated under reduced pressure to a relative density of 1.16 - 1.20 (80±5°C) or 50% of the corresponding sugar degree to obtain the concentrated solution; the concentrated solution was introduced into the alcohol precipitation tank, and the alcohol content was adjusted to 68% by adding an appropriate amount of ethanol, the mixture was left to stand for 20 hours until a complete precipitation status, the supernatant was separated by discarding the precipitate; the supernatant was concentrated to obtain the extract, which was dried to obtain Salvia miltiorrhiza extract.

The Salvia miltiorrhiza and Panax notoginseng extract was measured by aforementioned detection method, wherein, Danshensu, salvianolic acid T, protocatechuic aldehyde, salvianolic acid D, rosmarinic acid, salvianolic acid B, salvianolic acid A, notoginsenoside R1, ginsenoside Rg1, ginsenoside Re, ginsenoside Rb1, ginsenoside Rd, dihydrotanshinone I, tanshinone I, cryptotanshinone, and tanshinone IIA that were contained in the Salvia miltiorrhiza and Panax notoginseng extract were 20mg/g, 5mg/g, 10mg/g, 2mg/g, 0.2mg/g, 5mg/g, 5mg/g, 2mg/g, 1mg/g, 1mg/g, 10mg/g, 1mg/g, 0.1mg/g, 0.5mg/g, 0.2mg /g, and 1 mg/g, respectively.

90g of the above Salvia miltiorrhiza and Panax notoginseng extract and 10g of borneol were mixed evenly to obtain the traditional Chinese medicine composition.

### Example 9

500g of the raw material of Salvia miltiorrhiza was cut into pieces of 1 ~ 2 cm long, 102g of the raw material of Panax notoginseng was pulverized into granules of 1 cm, and 2.5% of the total weight of the raw materials of sodium bicarbonate was weighed. The weighed raw materials of Salvia miltiorrhiza, Panax notoginseng and sodium bicarbonate were put into the extracting tank, 6 times the amount of process water was added to each tank, and heated to boil and kept boiling for about 2h, and the mixture was filtered, the dregs of decoction were extracted for the second time by adding 6 times the amount of water and heated to boil and was kept boiling for 1h, the mixture was filtered and the dregs of decoction were discarded; the extract was concentrated under reduced pressure to a relative density of 1.16 - 1.20 (80±5°C) or 48% of the corresponding sugar degree to obtain the concentrated solution; the concentrated solution was introduced into the alcohol precipitation tank, and the alcohol content was adjusted to 65% by adding an appropriate amount of ethanol, the mixture was left to stand for 24 hours until a complete precipitation status, the supernatant was separated by discarding the precipitate; the supernatant was concentrated to obtain the extract, which was dried to obtain Salvia miltiorrhiza extract.

The Salvia miltiorrhiza and Panax notoginseng extract was measured by aforementioned detection method, wherein, Danshensu, salvianolic acid T, protocatechuic aldehyde, salvianolic acid D, rosmarinic acid, salvianolic acid B, salvianolic acid A, notoginsenoside R1, ginsenoside Rg1, ginsenoside Re, ginsenoside Rb1, ginsenoside Rd, dihydrotanshinone I, tanshinone I, cryptotanshinone, and tanshinone IIA that were contained in the Salvia miltiorrhiza and Panax notoginseng extract were 60 mg/g, 20 mg/g, 30 mg, 10mg/g, 10mg/g, 20mg/g, 20mg/g, 10mg/g, 40mg/g, 5mg/g, 40mg/g, 10mg/g, 0.5mg/g, 1mg/g, 1mg/g, and 5 mg/g, respectively.

99.9g of the above Salvia miltiorrhiza and Panax notoginseng extract and 0.1g of borneol were mixed evenly to obtain the traditional Chinese medicine composition.

### The preparation of the pharmaceutical formulation of the present invention

### Example 10

0.5g of the traditional Chinese medicine composition described in any one of Examples 1-9 and 10.5g of PEG-6000 were mixed evenly, and heated to be dissolved, then moved in a dropping pill dripping tank, and the mixture was dropped into liquid paraffin of 6 ~ 8 °C, then the oil inside was removed to obtain 400 dropping pills.

### Example 11

0.5g of the traditional Chinese medicine composition described in any one of Examples 1-9 and 4.5g of glucose, 0.9g of sodium thiosulfate and 1ml of distilled water were mixed evenly and lyophilized, then packaged into 500 vials.

### Example 12

0.5g of the traditional Chinese medicine composition described in any one of Examples 1-9 and 5.5g of mannitol, 0.9g of calcium sodium edetate and 2ml of distilled water were mixed evenly and lyophilized, then packaged into 300 vials.

### Example 13

0.5g of the traditional Chinese medicine composition described in any one of Examples 1-9 and 50g of starch, 50g of sucrose were mixed evenly, granulated and compressed to obtain tablets.

### Example 14

0.5g of the traditional Chinese medicine composition described in any one of Examples 1-9 and 50g of starch, 50g of sucrose were mixed evenly, granulated and encapsulated to obtain capsules.

### The preparation of compound Salvia miltiorrhiza micro-dropping pill of the present invention

### Example 15

82.5g of the traditional Chinese medicine composition prepared in Example 1 and 165g of PEG-6000 were taken.
(1) Premixing step: the traditional Chinese medicine composition was premixed with water, and stirred in a heat preservation tank of 40±10°C for more than 60 min to keep the water content of the traditional Chinese medicine composition to be 13.0wt%, the traditional Chinese medicine composition premixture was obtained for the following steps;
(2) Material homogenizing step: PEG-6000 was first added in the homogenizing tank to be heated to 90°C to a dissolved status, then the traditional Chinese medicine composition premixture was added; low-speed homogenization (3200rpm) was used to mix them, then the homogenization speed was increased to 5000rpm to carry out the homogenization for 6 minutes after the previous mixing. During the homogenization process, the temperature of the material was kept at 80±5°C. Thus, a molten medicinal solution was obtained.
(3) Dripping step: the aforementioned molten medicinal solution was introduced to the dripper, the vibration frequency of the dripper was adjusted to 137Hz, the dripper temperature was kept at 80 °C, and the molten medicinal solution passed through the dripper in a pressurized mode (with a dripping pressure of 1.8Bar) and dripped out from the bottom of the dripper, and the dripping speed matches the speed of the material homogenization in Step (1);
(4) Condensation step: the medicinal liquid dropped in the cooling tube was cooled by employing the low temperature inert gas, the cooling temperature was -115±5°C, to make the dropped medicine liquid cooled into a status of solid dropping pills;
(5) Drying step: The fluidized drying was used to dry the dropping pill, after the material formed a satisfying fluid state in fluidizing bed, it was heated to 25°C for drying for 60min, then heated to 45°C of 30min, and then heated to 55°C for 30min, then it was cooled down to below 30°C for discharging. The water content of the dropping pills is controlled at 3.0-7.0wt% to obtain intermediate pellets;
(6) Coating step: the coating powder consumption was calculated according to coating feed amount and the prescription, Opadry of 4% weight of the pellets was prepared to a coating solution of 18wt% concentration and stirred for 45min. The inlet air temperature was set to 25°C, and the qualified pellets were introduced into the fluidizing bed, then the inlet air temperature was increased to 48°C, when the material temperature reached 38°C, the coating process was started. During the coating process, the temperature of the material was controlled at 35-45°C, after completing the coating process, the temperature was lowered to below 30°C for discharging, and the pellets were sieved to obtain the coated intermediate pellets. The increase in weight of the coated intermediate pills was controlled at 3.3±0.7wt%, and the water content was controlled at 3.0~7.0wt%;
(7) Capsule preparation and packaging step: the dropping pills with a particle diameter of 1.0mm ~ 2.0mm were used for capsule filling, and a complete 100% on-site weight checking was executed by a capsule weight checking equipment, then packaged into final product.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

### Example 16

Compound Salvia miltiorrhiza micro-dropping pills were prepared as in Example 15, except that the weight ratio of the traditional Chinese medicine composition to PEG-6000 was 1:5.

### Example 17

Compound Salvia miltiorrhiza micro-dropping pills were prepared as in Example 15, except that the weight ratio of the traditional Chinese medicine composition to PEG-6000 was 5:1.

### Example 18

82.5g of the traditional Chinese medicine composition prepared in Example 1 and 165g of a mixture of 1:1 of cyclodextrin and agar were used to prepare the micro-dropping pills, and the preparation method was as follows:
(1) Material homogenizing step: the traditional Chinese medicine composition and the mixture of 1:1 of cyclodextrin and agar as the dropping pill matrix were put into the homogenizing tank, and mixed evenly at 1000 rpm for 1 min, then the materials were homogenized at 3000rpm for 1min. In the homogenizing process, the temperature was kept at 60°C to obtain a molten medicinal solution;
(2) Dripping step: the aforementioned molten medicinal solution was introduced to the dripper, under the condition of the dripper temperature at 70°C, the vibration frequency of the dripper at 50Hz, and the dripping pressure of 1.8Bar, the molten medicinal solution dripped out from the dripper by its vibration, and the dripping speed matched the speed of the material homogenization in Step (1);
(3) Condensation step: the medicinal liquid dropped in the cooling tube was rapidly cooled in the cooling gas to make the dropped medicine liquid solidified into dropping pill pellets with a particle diameter of 0.2mm, the temperature of the cooling gas was 0°C.

### Example 19

82.5g of the traditional Chinese medicine composition prepared in Example 1 and 165g of a mixture of 1:1 of cyclodextrin and lactose were used to prepare the compound Salvia miltiorrhiza micro-dropping pills, and the preparation method was as follows:
(1) Material homogenizing step: the traditional Chinese medicine composition and the mixture of 1:1 of Arab gum and agar as the dropping pill matrix were put into the homogenizing tank, and mixed evenly at 1000 rpm for 1 min, then the materials were homogenized at 5000rpm for 200 min. In the homogenizing process, the temperature was kept at 100°C to obtain a molten medicinal solution;
(2) Dripping step: the aforementioned molten medicinal solution was introduced to the dripper, under the condition of the dripper temperature at 300°C, the vibration frequency of the dripper at 300Hz, and the dripping pressure of 4.0 Bar, the molten medicinal solution dripped out from the dripper by its vibration, and the dripping speed matched the speed of the material homogenization in Step (1);
(3) Condensation step: the medicinal liquid dropped in the cooling tube was rapidly cooled in the cooling gas to make the dropped medicine liquid solidified into dropping pill pellets with a particle diameter of 4.0mm, the temperature of the cooling gas was -150°C.

### Example 20

82.5g of the traditional Chinese medicine composition prepared in Example 1 and 165g of lactitol were used to prepare the compound Salvia miltiorrhiza micro-dropping pills, and the preparation method was as follows:
(1) Material homogenizing step: the traditional Chinese medicine composition and lactitol as the dropping pill matrix were put into the homogenizing tank, and mixed evenly at 2500 rpm for 100 min, then the materials were homogenized at 6000rpm for 50 min. In the homogenizing process, the temperature was kept at 80°C to obtain a molten medicinal solution;
(2) Dripping step: the aforementioned molten medicinal solution was introduced to the dripper, under the condition of the dripper temperature at 150°C, the vibration frequency of the dripper at 150Hz, and the dripping pressure of 2 Bar, the molten medicinal solution dripped out from the dripper by its vibration, and the dripping speed matched the speed of the material homogenization in Step (1);
(3) Condensation step: the medicinal liquid dropped in the cooling tube was rapidly cooled in the cooling gas to make the dropped medicine liquid solidified into dropping pill pellets with a particle diameter of 2mm, the temperature of the cooling gas was -100°C.
(4) Drying step: the fluidized drying was used to dry the dropping pill at 50°C for 2h, to obtain the dry dropping pill pellets;
(5) Coating step: the dry dropping pill pellets were coated in a fluidization bed, and the weight ratio of coating material to the dry dropping pill pellets was 1:25, and the coating solution concentration was 10wt%, the coating temperature was 40°C, to obtain the coated dropping pills, and the coating material was Opadry.

### Example 21

82.5g of the traditional Chinese medicine composition prepared in Example 1 and 165g of PEG-8000 were used to prepare the compound Salvia miltiorrhiza micro-dropping pills, and the preparation method was as follows:

The aforementioned traditional Chinese medicine composition powder was added with water, and stirred at 60°C of more than 10min, to obtain the traditional Chinese medicine composition premixture.
(1) Material homogenizing step: the traditional Chinese medicine composition and PEG-8000 as the dropping pill matrix were put into the homogenizing tank, and mixed evenly at 2500 rpm for 100 min, then the materials were homogenized at 6000rpm for 50 min. In the homogenizing process, the temperature was kept at 80°C to obtain a molten medicinal solution;
(2) Dripping step: the aforementioned molten medicinal solution was introduced to the dripper, under the condition of the dripper temperature at 150°C, the vibration frequency of the dripper at 150Hz, and the dripping pressure of 2 Bar, the molten medicinal solution dripped out from the dripper by its vibration, and the dripping speed matched the speed of the material homogenization in Step (1);
(3) Condensation step: the medicinal liquid dropped in the cooling tube was rapidly cooled in the cooling gas to make the dropped medicine liquid solidified into dropping pill pellets with a particle diameter of 2mm, the temperature of the cooling gas was -100°C.
(4) Drying step: the fluidized drying was used to dry the dropping pill at 50°C for 2h, to obtain the dry dropping pill pellets;
(5) Coating step: the dry dropping pill pellets were coated in a fluidization bed, and the weight ratio of coating material to the dry dropping pill pellets was 1:25, and the coating solution concentration was 10wt%, the coating temperature was 40°C, to obtain the coated dropping pills, and the coating material was shellac.

### Example 22

92g of the traditional Chinese medicine composition prepared in Example 1 and 270g of PEG-1000 were used to prepare the compound Salvia miltiorrhiza micro-dropping pills, and the preparation method was as follows:

The aforementioned traditional Chinese medicine composition powder was added with water, and stirred at 30°C of more than 10min, to obtain the traditional Chinese medicine composition premixture.
(1) Material homogenizing step: the traditional Chinese medicine composition and PEG-1000 were put into the homogenizing tank, and mixed evenly at 2500 rpm for 100 min, then the materials were homogenized at 6000rpm for 20 min. In the homogenizing process, the temperature was kept at 100°C to obtain a molten medicinal solution;
(2) Dripping step: the aforementioned molten medicinal solution was introduced to the dripper, under the condition of the dripper temperature at 70°C, the vibration frequency of the dripper at 100Hz, the dripping pressure at 1.0 Bar, the acceleration at 1G, and the dripping speed at 10 kg/h, the molten medicinal solution dripped out from the dripper by its vibration, and the dripping speed matched the speed of the material homogenization in Step (1);
(3) Condensation step: the dropped medicinal liquid was rapidly cooled in the cooling gas to make the dropped medicine liquid solidified into dropping pill pellets with a particle diameter of 2mm, the temperature of the cooling gas was -80°C.
(4) Drying step: the gradient heating drying method was used to dry the dropping pill at -20°C to form a fluidized state and dried at 15°C for 10min, 35°C for 10min, and 55°C for 30min, to obtain the dry dropping pill pellets;
(5) Coating step: the dry dropping pill pellets were coated in a fluidization bed, and the weight ratio of coating material to the dry dropping pill pellets was 1:25, and the coating solution concentration was 10wt%, the coating temperature was 40°C, to obtain the coated dropping pills, and the coating material was cellulose acetate phthalate.

### Example 23

105g of the traditional Chinese medicine composition prepared in Example 1 and 35g of a mixture of 1:1 of PEG-4000 and PEG-6000 were used to prepare the compound Salvia miltiorrhiza micro-dropping pills, and the preparation method was as follows:

The aforementioned traditional Chinese medicine composition powder was added with water, and stirred at 80°C of more than 10min, to obtain the traditional Chinese medicine composition premixture.
(1) Material homogenizing step: the traditional Chinese medicine composition and the mixture of 1:1 of PEG-4000 and PEG-6000 were put into the homogenizing tank, and mixed evenly at 2500 rpm for 100 min, then the materials were homogenized at 6000rpm for 80 min. In the homogenizing process, the temperature was kept at 80°C to obtain a molten medicinal solution;
(2) Dripping step: the aforementioned molten medicinal solution was introduced to the dripper, under the condition of the dripper temperature at 100°C, the vibration frequency of the dripper at 200Hz, the dripping pressure at 3.0 Bar, the acceleration at 20G, and the dripping speed at 40 kg/h, the molten medicinal solution dripped out from the dripper by its vibration, and the dripping speed matched the speed of the material homogenization in Step (1);
(3) Condensation step: the dropped medicinal liquid was rapidly cooled in the cooling gas to make the dropped medicine liquid solidified into dropping pill pellets with a particle diameter of 2mm, the temperature of the cooling gas was -120°C.
(4) Drying step: the gradient heating drying method was used to dry the dropping pill at 30°C to form a fluidized state and dried at 35°C for 10min, 55°C for 10min, and 100°C for 60min, to obtain the dry dropping pill pellets;
(5) Coating step: the dry dropping pill pellets were coated in a fluidization bed, and the weight ratio of coating material to the dry dropping pill pellets was 1:25, and the coating solution concentration was 10wt%, the coating temperature was 35°C, to obtain the coated dropping pills, and the coating material was methyl acrylate.

### Example 24

600g of the traditional Chinese medicine composition prepared in Example 1, 5g of borneol, and 600g of dropping pill matrix xylitol were taken.
(1) Material homogenizing step: the xylitol was first put into the homogenizing tank and heated to 90°C to a dissolved status, then the traditional Chinese medicine composition was added and mixed evenly to obtain a molten medicinal solution;
(2) Dripping step: the aforementioned molten medicinal solution was introduced to the dripper in a pressurized mode, the dripper was heated with steam jacket, under the condition of the dripper temperature at 40°C and the vibration frequency of the dripper at 50Hz, the molten medicinal solution passed through the dripper and dripped out from the bottom of the dripper;
(3) Condensation step: the medicinal liquid dropped in the cooling tube was cooled by employing the low temperature inert gas, the cooling temperature was -20°C, to make the dropped medicinal liquid cooled into a status of solid dropping pills;
(4) Drying and coating step: The fluidized drying was used to dry the dropping pill which was then executed a drug-loading coating process, the drying temperature was 75°C, to obtain the coated micro-dropping pills with a particle diameter of 0.2mm~1.0mm;
(5) Packaging step: the coated dropping pills were used for capsule filling, and a complete 100% on-site weight checking was executed by a capsule weight checking equipment, then packaged into final product, the particle diameter of the micro-dropping pills was 0.2~1.0mm.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

### Example 25

600g of the traditional Chinese medicine composition prepared in Example 1, 5g of borneol, and 3000g of dropping pill matrix PEG-6000 and PEG-4000 were taken.
(1) Material homogenizing step: PEG-6000 and PEG-4000 were first put into the homogenizing tank and heated to 120°C to a dissolved status, then the traditional Chinese medicine composition was added and mixed evenly to obtain a molten medicinal solution;
(2) Dripping step: the aforementioned molten medicinal solution was introduced to the dripper in a pressurized mode, the dripper was heated with steam jacket, under the condition of the dripper temperature at 80°C and the vibration frequency of the dripper at 20Hz, the molten medicinal solution passed through the dripper and dripped out from the bottom of the dripper;
(3) Condensation step: the medicinal liquid dropped in the cooling tube was cooled by employing the low temperature inert gas, the cooling temperature was -80°C, to make the dropped medicinal liquid cooled into a status of solid dropping pills;
(4) Drying and coating step: The fluidized drying was used to dry the dropping pill which was then executed a drug-loading coating process, the drying temperature was 150°C, to obtain the coated micro-dropping pills with a particle diameter of 0. 5mm~1.0mm;
(5) Packaging step: the coated dropping pills were used for capsule filling, and a complete 100% on-site weight checking was executed by a capsule weight checking equipment, then packaged into final product.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

### Example 26

600g of the traditional Chinese medicine composition prepared in Example 1, 5g of borneol, and 120g of dropping pill matrix PEG-1000 were taken.
(1) Material homogenizing step: PEG-1000 was first put into the homogenizing tank and heated to 40°C to a dissolved status, then the traditional Chinese medicine composition was added and mixed evenly to obtain a molten medicinal solution;
(2) Dripping step: the aforementioned molten medicinal solution was introduced to the dripper in a pressurized mode, the dripper was heated with steam jacket, under the condition of the dripper temperature at 40~60°C and the vibration frequency of the dripper at 200Hz, the molten medicinal solution passed through the dripper and dripped out from the bottom of the dripper;
(3) Condensation step: the medicinal liquid dropped in the cooling tube was cooled by employing the low temperature inert gas, the cooling temperature was -100°C, to make the dropped medicinal liquid cooled into a status of solid dropping pills;
(4) Drying and coating step: The fluidized drying was used to dry the dropping pill which was then executed a drug-loading coating process, a fluidized state was formed at 30°C and dried at 25°C for 60min, 45°C for 30min, and 55°C for 30min, obtain the coated micro-dropping pills with a particle diameter of 3.0mm~4.0mm;
(5) Packaging step: the coated dropping pills were used for capsule filling, and a complete 100% on-site weight checking was executed by a capsule weight checking equipment, then packaged into final product.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

### Example 27

600g of the traditional Chinese medicine composition prepared in Example 1, 5g of borneol, and 3000g of dropping pill matrix PEG-6000 and PEG-4000 were taken.
(1) Material homogenizing step: PEG-6000 and PEG-4000 were first put into the homogenizing tank and heated to 120°C to a dissolved status, then the traditional Chinese medicine composition was added and mixed evenly at 1000rpm for 1 min, then homogenized at 3000rpm for1 min, to obtain a molten medicinal solution;
(2) Dripping step: the aforementioned molten medicinal solution was introduced to the dripper in a pressurized mode, the dripper was heated with steam jacket, under the condition of the dripper temperature at 70°C, the vibration frequency of the dripper at 50Hz, and the dripping pressure at 0.5Bar, the molten medicinal solution passed through the dripper and dripped out from the bottom of the dripper;
(3) Condensation step: the medicinal liquid dropped in the cooling tube was cooled by employing the low temperature inert gas, the cooling temperature was 0°C, to make the dropped medicinal liquid cooled into a status of solid dropping pills;
(4) Drying and coating step: The fluidized drying was used to dry the dropping pill which was then executed a drug-loading coating process, the drying temperature was 150°C, to obtain the coated micro-dropping pills with a particle diameter of 0.2mm;
(5) Packaging step: the coated dropping pills were used for capsule filling, and a complete 100% on-site weight checking was executed by a capsule weight checking equipment, then packaged into final product.

### Example 28

600g of the traditional Chinese medicine composition prepared in Example 1, 5g of borneol, and 1800g of dropping pill matrix PEG-6000 were taken.
(1) Material homogenizing step: PEG-6000 was first put into the homogenizing tank and heated to 120°C to a dissolved status, then the traditional Chinese medicine composition was added and mixed evenly at 5000rpm for 200 min, then homogenized at 10000rpm for 1 min, to obtain a molten medicinal solution;
(2) Dripping step: the aforementioned molten medicinal solution was introduced to the dripper in a pressurized mode, the dripper was heated with steam jacket, under the condition of the dripper temperature at 300°C, the vibration frequency of the dripper at 300Hz, and the dripping pressure at 4.0Bar, the molten medicinal solution passed through the dripper and dripped out from the bottom of the dripper;
(3) Condensation step: the medicinal liquid dropped in the cooling tube was cooled by employing the low temperature inert gas, the cooling temperature was -150°C, to make the dropped medicinal liquid cooled into a status of solid dropping pills;
(4) Drying and coating step: The fluidized drying was used to dry the dropping pill which was then executed a drug-loading coating process, the drying temperature was 150°C, to obtain the coated micro-dropping pills with a particle diameter of 4.0mm;
(5) Packaging step: the coated dropping pills were used for capsule filling, and a complete 100% on-site weight checking was executed by a capsule weight checking equipment, then packaged into final product.

### Example 29

600g of the traditional Chinese medicine composition prepared in Example 1, 5g of borneol, and 2400g of dropping pill matrix PEG-4000 were taken.
(1) Material homogenizing step: PEG-4000 was first put into the homogenizing tank and heated to 120°C to a dissolved status, then the traditional Chinese medicine composition was added and mixed evenly at 3000rpm for 10 min, then homogenized at 4000rpm for 5 min, during the homogenizing process, the temperature of the materials were kept at 70~90°C, to obtain a molten medicinal solution;
(2) Dripping step: the aforementioned molten medicinal solution was introduced to the dripper in a pressurized mode, the dripper was heated with steam jacket, under the condition of the dripper temperature at 70°C, the vibration frequency of the dripper at 90Hz, and the dripping pressure at 1.0Bar, the molten medicinal solution passed through the dripper and dripped out from the bottom of the dripper;
(3) Condensation step: the medicinal liquid dropped in the cooling tube was cooled by employing the low temperature inert gas, the cooling temperature was -140°C, to make the dropped medicinal liquid cooled into a status of solid dropping pills;
(4) Drying step: The fluidized drying was used to dry the dropping pills, the drying temperature was 150°C, to obtain the coated micro-dropping pills with a particle diameter of 1.0mm.

### Example 30

600g of the traditional Chinese medicine composition prepared in Example 1, 5g of borneol, and 2400g of dropping pill matrix PEG-4000 were taken.
(1) Material homogenizing step: PEG-4000 was first put into the homogenizing tank and heated to 120°C to a dissolved status, then the traditional Chinese medicine composition was added and mixed evenly at 4000rpm for 60 min, then homogenized at 9000rpm for 30 min, during the homogenizing process, the temperature of the materials were kept at 90°C, to obtain a molten medicinal solution;
(2) Dripping step: the aforementioned molten medicinal solution was introduced to the dripper in a pressurized mode, the dripper was heated with steam jacket, under the condition of the dripper temperature at 100°C, the vibration frequency of the dripper at 200Hz, and the dripping pressure at 3.0Bar, the molten medicinal solution passed through the dripper and dripped out from the bottom of the dripper;
(3) Condensation step: the medicinal liquid dropped in the cooling tube was cooled by employing the low temperature inert gas, the cooling temperature was -140°C, to make the dropped medicinal liquid cooled into a status of solid dropping pills;
(4) Drying step: The fluidized drying was used to dry the dropping pills, the drying temperature was 150°C, to obtain the coated micro-dropping pills with a particle diameter of 2.0mm.

### Example 31

600g of the traditional Chinese medicine composition prepared in Example 1, 5g of borneol, and 2000g of dropping pill matrix PEG-6000 were taken.
(1) Material homogenizing step: PEG-6000 was first put into the homogenizing tank and heated to 90°C to a dissolved status, then the traditional Chinese medicine composition was added and mixed evenly, to obtain a molten medicinal solution;
(2) Dripping step: the aforementioned molten medicinal solution was introduced to the dripper in a pressurized mode, the dripper was heated with steam jacket, under the condition of the dripper temperature at 80°C, and the vibration frequency of the dripper at 50Hz, the molten medicinal solution passed through the dripper and dripped out from the bottom of the dripper;
(3) Condensation step: the medicinal liquid dropped in the cooling tube was cooled by employing the low temperature inert gas, the cooling temperature was -20°C, to make the dropped medicinal liquid cooled into a status of solid dropping pills;
(4) Drying and coating step: The fluidized drying was used to dry the dropping pill which was then executed a drug-loading coating process, the drying temperature was 75°C, to obtain the coated micro-dropping pills with a particle diameter of 1.0∼2.0mm;
(5) Packaging step: the coated dropping pills were used for capsule filling, and a complete 100% on-site weight checking was executed by a capsule weight checking equipment, then packaged into final product.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

### Example 32

82.5g of the traditional Chinese medicine composition prepared in Example 1 and 165g of PEG-6000 were taken.
(1)Material homogenizing step: PEG-6000 was first added in the homogenizing tank to be heated to 90°C to a dissolved status, then the traditional Chinese medicine composition was added; low-speed homogenization (3200rpm) was used to mix them, then the homogenization speed was increased to 5000rpm to carry out the homogenization for 6 minutes after the previous mixing. During the homogenization process, the temperature of the material was kept at 80±5°C. Thus, a molten medicinal solution was obtained.
(2) Dripping step: the aforementioned molten medicinal solution was introduced to the dripper, the vibration frequency of the dripper was adjusted to 137Hz, the dripper temperature was kept at 80 °C, and the molten medicinal solution passed through the dripper in a pressurized mode (with a dripping pressure of 1.8Bar) and dripped out from the bottom of the dripper, and the dripping speed matches the speed of the material homogenization in Step (1);
(3) Condensation step: the medicinal liquid dropped in the cooling tube was cooled by employing the low temperature inert gas, the cooling temperature was -115±5°C, to make the dropped medicine liquid cooled into a status of solid dropping pills;
(4) Drying step: The fluidized drying was used to dry the dropping pill, after the material formed a satisfying fluid state in fluidizing bed, it was heated to 25°C for drying for 60min, then heated to 45°C of 30min, and then heated to 55°C for 30min, then it was cooled down to below 30°C for discharging. The water content of the dropping pills is controlled at 3.0-7.0wt% to obtain intermediate pellets;
(5) Coating step: the coating powder consumption was calculated according to coating feed amount and the prescription, Opadry of 4% weight of the pellets was prepared to a coating solution of 18wt% concentration and stirred for 45min. The inlet air temperature was set to 25°C, and the qualified pellets were introduced into the fluidizing bed, then the inlet air temperature was increased to 48°C, when the material temperature reached 38°C, the coating process was started. During the coating process, the temperature of the material was controlled at 35-45°C, after completing the coating process, the temperature was lowered to below 30°C for discharging, and the pellets were sieved to obtain the coated intermediate pellets. The increase in weight of the coated intermediate pills was controlled at 3.3±0.7wt%, and the water content was controlled at 3.0~7.0wt%;
(6) Capsule preparation and packaging step: the dropping pills with a particle diameter of 1.0mm ~ 2.0mm were used for capsule filling, and a complete 100% on-site weight checking was executed by a capsule weight checking equipment, then packaged into final product.

Wherein, in the dripping process, the dropping pill forming situation was monitored in a real-time onsite monitoring and adjustment mode by the stroboscopic irradiation and the visual observation; after the dropping pills were coated, a sieving step could be added to improve the pellet size uniformity and roundness.

As a result of the inventors' research and efficacy tests, it was found that the micro-dropping pills obtained in Examples 15-32 have similar beneficial effects such as good efficacy, high bioavailability, small dose of drug required by the patients, and good compliance, compared to the existing compound Salvia miltiorrhiza dropping pills.

### Drug efficacy examples

Drug efficacy example 01 The effect of the product of Example 32 on the mortality rate in the LPS-induced DIC model rats

### 1. Experimental materials and methods

### 1.1 Experimental animals

SD rats, SPF grade, male, 220-250 g. Animal certificate number: 110011200105605444.

### 1.2 Test substance

The product obtained according to Example 32 (hereinafter referred to as micro-dropping pill)

### 1.3 Dose design

The dose administered to rats in this experiment was 837 mg micro-dropping pills/kg.

### 1.4 Experimental method

Drug preparation method: 837mg of micro-dropping pills were precisely weighed, placed in a container, and a suitable amount of pure water was added to completely dissolve the micro-dropping pills under ultrasound condition, the final volume was adjusted to 10ml.

Experimental method: Fourteen rats (n=7) were randomly divided into the model group and the micro-dropping pill group according to the references and previous experimental studies. After the experimental animals in each group were weighed, the DIC model was replicated by tail vein injection of 30 mg/kg LPS according to body weight. The model group was injected with 30mg/kg LPS in tail vein, and the micro-dropping pill group was administered by gavage at 20 min after replication of the model according to the body weight of each rat, and the administered dose was 837mg/kg and the administered volume was 10ml/kg. The mortality rates of experimental animals in each group were observed until 72h after replication of the model.

### 2. Experimental results

The influence of micro-dropping pills on mortality rates of LPS-induced DIC model rats

The experimental results showed that the mortality rates of the model group were 57.1% with tail vein 30 mg/kg LPS and 14.3% with oral administration of micro-dropping pills 837 mg micro-dropping pills/kg, observed up to 72 h after replication of the model.

**Table 3 Effects of Micro-dropping Pills on Mortality Rates of LPS-induced DIC Model Rats**

| Group | Animal number | Observation time (after replication of the model) | | | | |
|---|---|---|---|---|---|---|
| | | 6h | 24h | 48h | 72h | Mortality Rates |
| Model group | 7 | 1 | 3 | - | - | 57.1% |
| Micro-dropping pill group | 7 | - | 1 | - | - | 14.3% |

### 3. Conclusions

Under the present experimental conditions, single oral administration of the micro-dropping pills of the present invention after replication of the model significantly increased the survival rate of LPS-induced SD rats.

Drug efficacy example 02 The effect of the product of Example 32 on the mortality rate in the LPS-induced DIC model mice

### 1. Experimental materials and methods

### 1.1 Experimental animals

Kunming mice, SPF grade, male, 18-22 g. Animal certificate number: 110011200107075313.

### 1.2 Test substance

The product obtained according to Example 32 (hereinafter referred to as micro-dropping pill)

### 1.3 Dosage design

The dose administered to mice in this experiment was 1660.5 mg micro-dropping pills/kg.

### 1.4 Experimental method

Drug preparation method: 1660.5mg of micro-dropping pills were precisely weighed, placed in a container, and a suitable amount of pure water was added to completely dissolve the micro-dropping pills under ultrasound condition, the final volume was adjusted to 10ml.

Experimental method: Fourteen Kunming mice (n=8) were randomly divided into the model group and the micro-dropping pill group. After the experimental animals in each group were weighed, the DIC model was replicated by intraperitoneal injection of 35 mg/kg LPS according to the body weight. The model group was injected intraperitoneally with 35mg/kg LPS, and the micro-dropping pill group was administered by gavage at 20 min after replication of the model according to the body weight of each mouse, and the administered dose was 1660.5mg/kg and the administered volume was 10ml/kg. The mortality rates of experimental animals in each group were observed up to 72h after replication of the model.

### 2. Experimental results

The influence of micro-dropping pills on mortality rates of LPS-induced DIC model mice

The experimental results showed that the mortality rates of the model group were 87.5% when 35mg/kg LPS was injected intraperitoneally and 12.5% when 1660.5mg of micro-dropping pills/kg was given orally until 72h after replication of the model.

**Table 4 Effects of Micro-dropping Pills on Mortality Rates of LPS-induced DIC Model Mice**

| Group | Animal number | Observation time (after replication of the model) | | | | |
|---|---|---|---|---|---|---|
| | | 6h | 24h | 48h | 72h | Mortality Rates |
| Model group | 8 | - | 4 | 3 | - | 87.5% |
| Micro-dropping pill group | 8 | - | - | 1 | - | 12.5% |

### 3. Conclusions

Under the present experimental conditions, single oral administration of the micro-dropping pills of the present invention after replication of the model significantly increased the survival rate of LPS-induced Kunming mice.

Drug efficacy example 03 The effect of the product of Example 32 on the mortality rate in the LPS-induced DIC model mice

### 1. Experimental materials and methods

### 1.1 Experimental animals

SD rats, SPF grade, male, 220-250 g. Animal certificate number: 110011200105605444.

### 1.2 Test substance

The product obtained according to Example 32 (hereinafter referred to as micro-dropping pill)

### 1.3 Dose design

The dose administered to rats in this experiment was 837 mg micro-dropping pills/kg.

### 1.4 Experimental method

Drug preparation method: 837mg of micro-dropping pills were precisely weighed, placed in a container, and a suitable amount of pure water was added to completely dissolve the micro-dropping pills under ultrasound condition, the final volume was adjusted to 10ml.

Experimental method: Nine rats (n=3) were randomly divided into the normal group, the model group, and the micro-dropping pill group. After the experimental animals in each group were weighed, the DIC model was replicated by tail vein injection of 5 mg/kg LPS according to body weight. The model group was injected with 5mg/kg LPS in tail vein, and the micro-dropping pill group was administered by gavage at 20 min after replication of the model according to the body weight of each rat, and the administered dose was 837mg/kg and the administered volume was 10ml/kg. The plasma was taken from each group of experimental animals 4h after replication of the model for PT test.

### 2. Experimental results

The influence of micro-dropping pills on prothrombin time (PT) of LPS-induced DIC model rats

The experimental results showed that the PT value of model rats was prolonged after 4h from tail vein injection of 5mg/kg LPS compared with the normal group, and oral administration of 837 mg micro-dropping pills/kg significantly improved the prolongation of PT time due to LPS.

**Table5 Effects of Micro-dropping Pills on PT Values in High-dose LPS-induced DIC Model Rats**

| Group | PT Value(s) |
|---|---|
| Normal group | 13.33±1.31 |
| Model group | 30.35±1.58^{∗∗} |
| Micro-dropping pill group | 17.35±0.35## |

| | |
|---|---|
| ^{∗∗}: compared with normal group, P<0.01; ##: compared with model group, P<0.01 | |

### 3. Conclusions

Under the present experimental conditions, the PT values of rats in the model group were prolonged compared with the normal group, and the oral administration of 837mg of micro-dropping pill /kg of the present invention significantly improved the prolongation of PT time due to LPS.

### Drug Efficacy Example 04: Effect of the product of Example 32 on systemic diffuse coagulation index (D-Dimer) in patients with severe novel coronavirus pneumonia

The following efficacy trial is a clinical trial conducted in a humanitarian spirit under the conditions permitted by the clinical approval of the US FDA. The enrollment criteria were, primarily, patients hospitalized with severe novel coronavirus pneumonia and with significant systemic circulatory disturbances and diffuse coagulation states. The medical value of the invention in the treatment of novel coronavirus pneumonia was reflected by the addition of 300 mg/dose of the product prepared according to **Example 32**, three times daily, while the patient maintained the necessary medical conditions, and by the improvement in the length of stay and the decrease in the systemic diffuse coagulation index D-Dimer.

**Table 6 Table of Changes in D-Dimers**

| Subject | 1 | 2 | 3 | 4 | 5 |
|---|---|---|---|---|---|
| Day 1 | - | 1.4 | 0.93 | 1.21 | 1.36 |
| Day 2 | 0.79 | 1.02 | 0.97 | 0.56 | 1.15 |
| Day 3 | 0.79 | Drug withdrawal | 0.81 | 0.59 | * |
| Day 4 | 0.52 | | 0.52 | * | |
| Day 5 | | | | * | |
| Day 6 | | | | | |

| | | | | | |
|---|---|---|---|---|---|
| ^{∗}Indicates that online data is not available. | | | | | |

No patients were exacerbated or transferred to the ICU in this trial (usually the ratio is 4%);
No patient died in this trial (usually the ratio is 2%);
The average length of stay in this trial was 2.1 days (usually 7 days).

In summary, the average length of stay in the patients enrolled in this trial decreased from seven days in the control group to two days, with a significant decrease in biochemical D-Dimer, and improvement in systemic diffuse coagulation, except for one patient who fell off. To date, none of the patients have deteriorated, been admitted to the emergency ward, or died, demonstrating the clinical value of the present invention in the treatment of novel coronavirus pneumonia.

## Claims

1. An application of a traditional Chinese medicine composition in the preparation of a medicament for preventing and/or treating novel coronavirus pneumonia, wherein: the traditional Chinese medicine composition is composed of 50.0%∼99.9% by weight of Salvia miltiorrhiza and Panax notoginseng extract and 0.1%∼50.0% by weight of borneol, wherein the Salvia miltiorrhiza and Panax notoginseng extract contains the following components, the weight ratios of each component are:
Danshensu: salvianolic acid T: protocatechuic aldehyde: salvianolic acid D: rosmarinic acid: salvianolic acid B: salvianolic acid A:Notoginsenoside R1: ginsenoside Rg1: ginsenoside Re: ginsenoside Rb1: ginsenoside Rd: Dihydrotanshinone I: tanshinone I: cryptotanshinone: tanshinone IIA = (2∼6): (0.5∼2): (1∼3): (0.2∼1): (0.2∼1): (0.5∼2): (0.5∼2): (0.2∼1): (1∼4): (0.1∼0.5): (1∼4): (0.1∼1): (0.01∼0.05): (0.05∼0.1): (0.02∼0.1): (0.1∼0.5).

2. The application according to claim 1, wherein, it can prevent or reduce the formation of thrombus in patients having novel coronavirus pneumonia.

3. The application according to claim 1, wherein, it can improve the microcirculation disturbance in patients having novel coronavirus pneumonia.

4. The application according to claim 1, wherein the disseminated intravascular coagulation state of patients having novel coronavirus pneumonia can be inhibited, thus the mortality rate can be reduced.

5. The application according to claim 1, wherein, the novel coronavirus pneumonia is common, or moderate and severe pneumonia.

6. The application according to claim 1, wherein the traditional Chinese medicine composition is composed of 75.0∼99.9% by weight of Salvia miltiorrhiza and Panax notoginseng extract and 0.1%~25.0% by weight of borneol.

7. The application of any one of claims 1-6, wherein the traditional Chinese medicine composition is made into a pharmaceutically acceptable formulation.

8. The application according to claim 7, wherein, the formulation is dropping pill or micro-dropping pill, preferably micro-dropping pill, and the micro-dropping pill is prepared from the traditional Chinese medicine composition and the dropping pill matrix in a weight ratio of 1:5 ~5:1.

9. The application according to claim 8, wherein the preparation method of the micro-dropping pill comprises the following steps:
(1) Material homogenizing step: put the raw materials and dropping pill matrix into the homogenizing tank, and mix evenly at 1000∼5000 rpm for 1∼200 min, then homogenize the raw materials at 3000∼10000rpm for 1∼100min. In the homogenizing process, the temperature is maintained at 60 ~ 100°C to obtain a molten medicinal solution, and the weight ratio of the raw material to the dropping pill matrix is 1:5 ~ 5:1;
(2) Dripping step: transfer the above molten medicinal solution to the dripper, under the conditions of dripper temperature at 70∼300°C, dripping vibration frequency at 2∼2000 Hz, dripping pressure at 0.5∼4.0 Bar, and acceleration at 1~20 G, micro-dropping pills are made by vibration of the dripper, and the dripping speed matches the speed of the material homogenizing in Step (1);
(3) Condensation step: the droplets are rapidly cooled in a cooling gas, and solidified into solid dropping pills with a particle size of 0.2 mm~4.0 mm, and the temperature of the cooling gas is below 0°C.

10. The application according to claim 9, wherein, in the above Step (1), the dropping pill matrix includes one or more combinations of PEGs, sorbitol, xylitol, lactitol, maltose, starch, methyl cellulose, sodium carboxymethyl cellulose, hydroxypropyl methyl cellulose, Arabic gum, alginic acid, dextrin, cyclodextrin, agar, lactose; the preferred dropping pill matrix is solid PEG, such as PEG-1000, PEG-2000, PEG-3000, PEG-4000 , PEG-5000, PEG-6000, PEG-7000, and PEG-8000, more preferably one or more combinations of PEG-1000, PEG-2000, PEG-3000, PEG-4000, PEG-6000, and PEG-8000 , most preferably PEG-6000, PEG-4000 or a combination of PEG-4000 and PEG-6000.

11. The application according to claim 9, wherein, the preparation method of the micro-dropping pill comprises the following steps:
(1) Material homogenizing step: put the raw material and dropping pill matrix into the homogenizing tank, and mix evenly at 1000~5000rpm, and then homogenize the material at 3000~10000rpm for 20∼80 min. During the homogenizing process, keep the temperature at 80 ~ 100°C to obtain a molten medicinal solution, and the weight ratio of raw materials to the dropping pill matrix is 1:3 ~ 3:1;
(2) Dripping step: transfer the above molten medicinal solution to the dripper, under the conditions of dripper temperature at 70~200°C, dripping vibration frequency at 20∼300 Hz, dripping pressure at 0.5∼4.0 Bar, and acceleration at 1~15 G, micro-dropping pills are made by vibration of the dripper, and the dripping speed matches the speed of the material homogenizing in Step (1);
(3) Condensation step: the droplets are rapidly cooled in a cooling air, and solidified into solid dropping pills with a particle size of 0.2 mm~4.0 mm, and the temperature of the cooling air is below 0°C.

12. The application according to claim 11, wherein the preparation method further comprises a drying step as Step (4), in which the pills are dried by fluidized drying equipment at -20~100°C, preferably -20~90°C for 1~4 hours, to obtain plain pellets.

13. The application according to claim 12, wherein, Step (4) adopts a gradient heating drying method: forming a fluidized state at -20~30°C, drying at 15~35°C for 10~120min, drying at 35~55°C for 10~60min, and drying at 55~100°C °C for 0~60min; preferably, the gradient heating drying method is carried out as follows: forming a fluidized state at 0~20°C, drying at 25°C for 60min, drying at 45°C for 30min, and drying at 55°C for 0~30min.

14. The application according to claim 13, wherein, the preparation method further comprises a coating step as Step (5), in which the plain pellets obtained in Step (4) in a fluidized state are coated at a temperature of 30-65°C; the coating liquid concentration is 5~25wt%, preferably 18∼20wt%, wherein, the coating material is selected from: shellac, cellulose acetate phthalate, methyl acrylate, methyl methacrylate or Opadry; the weight ratio of the coating material to the plain pellets is 1:50∼1:10, preferably 1:50∼1:25.

15. The application according to any one of claims 10 to 14, wherein, before Step (1), the preparation method may further comprise a material premixing step, after adding water to the medicinal extract or powder, stirring at 30~80°C for more than 10 min to obtain a medicinal premixture.
